# EUROPEAN PATENT SPECIFICATION

(11) **EP 1 633 426 B1**
(45) Date of publication and mention of the grant of the patent: **27.01.2010**
(21) Application number: 04752917.7
(22) Date of filing: 21.05.2004
(51) Int. Cl.: A61M 31/00, A61F 11/00, A61L 29/16, A61M 25/00

(54) **THERAPEUTIC AGENT DELIVERY**
ABGABE EINES THERAPEUTISCHEN MITTELS
APPORT D'AGENT THERAPEUTIQUE

(30) Priority: 22.05.2003 US 444213
(43) Date of publication of application: 15.03.2006
(73) Proprietor: Atrium Medical Corporation, Hudson, NH 03051 (US)
(72) Inventor: HERWECK, Steve, A., Nashua, NH 03062 (US); MARTAKOS, Paul, Pelham, NH 03076 (US)
(74) Representative: Riem, Charles Hendrik
(86) International application number: PCT/US2004/015993
(87) International publication number: WO 2004/105833

(56) References cited:
- WO-A-00/43051
- WO-A-94/05361
- WO-A2-01/28606
- WO-A2-03/009883
- US-A- 5 286 254
- US-A- 5 735 897
- US-A- 5 795 318
- US-A- 5 868 704
- US-A- 5 972 027
- US-A1- 2002 133 224
- US-A1- 2003 068 355
- US-B1- 6 206 914

## Description

### FIELD OF THE INVENTION

The present invention relates to therapeutic agent delivery, and more particularly to a device and/or system for delivering a multi-part therapeutic application to a targeted location within a patient.

### BACKGROUND OF THE INVENTION

Radially expandable devices are utilized in a wide range of applications including a number of biological applications. Radially expandable devices in the form of inflatable balloons have been proposed for treatment of body passages occluded by disease and for maintenance of the proper position of catheter delivered medical devices within such body passages. Such expandable devices can be constructed of elastomeric materials such as latex.

Some elastomeric balloons are made to deliver a liquid or gas that includes a drug, to a targeted location. Unfortunately, the range of drugs that may be delivered via such balloons is somewhat limited. The only therapeutic drugs that are currently available for use with an elastomeric balloon are those that are pre-mixed or require no mixing but can be stored for a predetermined shelf life. In other words, the therapeutic drugs that are currently available must be drugs that can be made by a manufacturer, possibly stored in a manufacturer storage facility, shipped to a clinical user, stored by the clinical user for a period of time, and then finally utilized when needed. Such a distribution process can take from a few days to a few months. Drugs that can withstand such a process can be either more expensive because of preservatives and temperature safeguards that must be added, or are otherwise less desirable because certain drug characteristics cannot be taken advantage of if the drug must be able to endure such a process.

In addition, therapeutic drugs that might only exist in a fluid form for a limited period (such as a few minutes or hours) are also precluded from use in existing catheter balloon systems. This is because their transformation to a non-fluid (or highly viscous) form, or some other transition to a less useful form, occurs well before they can be shipped to a clinical user and introduced to a catheter balloon. Such drugs can create the potential for doctors, nurses, or clinical technicians mistakenly administering expired medications which could be either ineffective or harmful.

US Patent No. 6,500,174 describes a medical balloon catheter assembly including a balloon with a permeable region and a non-permeable region. The permeable region is formed from a porous material that allows a volume of pressurized fluid to pass from within a chamber formed by the balloon and into the permeable regions sufficiently such that the fluid may be ablatively coupled to tissue engaged by the permeable region. The assembly includes an ablation element disposed within the chamber of the balloon, which generates the required ablative electrical current for translation through the pressurized fluid to the tissue external to the chamber. Thus, the structure disclosed is sufficient merely to allow fluid to pass from inside the balloon chamber to outside the balloon chamber, through the permeable region, as previously described in the other elastomeric balloons made to deliver a liquid or gas. However, the ablative assembly does not provide a user with the ability to combine multiple fluids using the drug delivery apparatus to result in a mixture therapeutic agent. In addition, there is no provision for maintaining a pressure in the fluid after the fluid passes through the permeable region to improve tissue absorption of the fluid.

US Patent No. 6,491,938 describes methods for inhibiting stenosis or retenosis following vascular trauma, comprising administering an effective amount of cytoskeletal inhibitor. The patent describes a kit comprising a device adapted for the local delivery of at least two therapeutic agents, a unit dosage of a first therapeutic agent, and a unit dosage of a second therapeutic agent, along with instructions as to their usage. The unit dosage forms of the first and second agents may be introduced via discrete lumens of a catheter, or mixed together prior to introduction into a single lumen or catheter. However, there is no discussion or structure disclosed concerning maintaining at least two different components in separate storage devices to be combined to form the therapeutic agent or other desired agent. The '938 patent merely discusses applying multiple different agents to a body tissue as performed by other known elastomeric balloons made to deliver a liquid or gas. Such known balloons simply receive multiple agents through the catheter or catheters for delivery through the balloon. In addition, the '938 patent does not disclose or discuss the ability of the balloon structure to maintain a fluid pressure external to the balloon as the fluid is applied to the tissue to improve localized therapeutic agent or drug permeation into the targeted tissue and reduce the volume of systemic medication required for effective drug application or therapeutic result.

### SUMMARY OF THE INVENTION

There is a need in the art for a therapeutic agent delivery system for combining multiple components to form a therapeutic agent and deliver the agent to a targeted location within a patient. The present invention is directed toward further solutions to address this need.

Interesting embodiments of the invention are the following.

In an embodiment, a therapeutic agent delivery system comprises a non-perforated irrigating shaped form in fluid communication with a first agent source. The irrigating shaped form is sized and dimensioned for positioning within a patient's body. The system also has a second agent disposed in a coating on the irrigating shaped form. The non-perforated irrigating shaped form has a microporous structure extending between an inner wall and an outer wall of the non-perforated irrigating shaped form. The non-perforated irrigating shaped form is sized and dimensioned for positioning within a patient's body against a targeted location. Upon delivery of a first agent from the first agent source permeating through the microporous structure of the irrigating shaped form, the first agent reacts with the second agent forming the therapeutic agent on a portion of the irrigating shaped form for a desired dwell time.

In a further embodiment, a fluid pressure and dwell time of the therapeutic agent delivery system are controllable to vary permeability of the therapeutic agent into the targeted location.

In a further embodiment of the therapeutic agent delivery system, the irrigating shaped form is structurally suitable for pressurization at about 6 atmospheres.

In a further embodiment, a physical state if the therapeutic agent comprises at least one of a gas, liquid, powder, gel, micro-particle, and nano-particle.

In a further embodiment, the first agent and the second agent have substantially similar viscosity.

In a further embodiment, the therapeutic agent emits to the targeted location under pressure and maintains fluid pressure external to the irrigating shaped form for the desired dwell time.

In a further embodiment, the dwell time is controllable to vary a dosage of the therapeutic agent applied to the targeted location.

In a further embodiment, the non-perforated irrigating shaped form has a lumen extending along a longitudinal axis of the form; and the non-perforated irrigating shaped form is radially expandable with respect to the axis.

In a further embodiment, the non-perforated irrigating shaped form is made from an inelastic material.

In a further embodiment of the therapeutic agent delivery system, the irrigating shaped form is coupled with the first agent source.

Interesting aspects and advantages are discussed herein below.

In accordance with one example embodiment of the present invention, a therapeutic agent delivery system includes a non-perforated irrigating shaped form in fluid communication with a first agent source, the irrigating shaped form sized and dimensioned for positioning within a patient's body. A second agent is disposed on the irrigating shaped form. Upon delivery of a first agent from the first agent source through the irrigating shaped form with the irrigating shaped form pressed against a targeted location, the first agent reacts with the second agent forming the therapeutic agent emitted from a portion of the irrigating shaped form at the targeted location for a desired dwell time.

In accordance with various aspects of the present invention, the irrigating shaped form is coupled with the first agent source. The fluid pressure and dwell time of the therapeutic agent delivery system are controllable to vary permeability of the therapeutic agent into the targeted location.

In accordance with further aspects of the present invention, the structure of the irrigating shaped form is suitable for pressurization at about 6 atmospheres. A physical state of the therapeutic agent can include at least one of a gas, liquid, powder, gel, micro-particle, and nano-particle. The first agent and the second agent can have substantially similar viscosity. The therapeutic agent can emit to the targeted location under pressure and maintain fluid pressure external to the irrigating shaped form for the desired dwell time.

In accordance with an example embodiment of the present invention, a therapeutic agent delivery system includes a first agent source, a second agent source, and a non-perforated irrigating shaped form. The non-perforated irrigating shaped form is positioned within a patient's body and in fluid communication with the first agent source and the second agent source. Upon introduction of a first agent to the irrigating shaped form, the first agent reacts with the second agent forming the therapeutic agent for emission to a targeted location within the patient's body.

In accordance with various aspects of the present invention, the irrigating shaped form is coupled with the first agent source. The drug delivery structure can apply a fluid pressure against the targeted location in a controlled manner for a desired dwell time, to effect a concentration of the therapeutic agent as the therapeutic agent is applied to the targeted location. The fluid pressure and dwell time can be controlled to vary a rate of therapeutic agent permeation into the targeted location. The irrigating shaped form can be suitable for pressurization at about six atmospheres. The physical state of the therapeutic agent can include at least one of a gas, liquid, powder, gel, micro-particle, and nano-particle. The therapeutic agent can emit to the targeted location under pressure and maintain fluid pressure external to the irrigating shaped form for a desired dwell time.

In accordance with another embodiment of the present invention, a therapeutic agent delivery system includes a therapeutic agent delivery structure. A microporous film is disposed about at least a portion of the therapeutic agent delivery structure. A first agent source contains a first agent and able to be in fluid communication with the therapeutic agent delivery structure. A second agent source contains a second agent and is able to be in fluid communication with the therapeutic agent delivery structure. The therapeutic agent delivery structure is suitable for applying a pressure to a targeted location within a patient's body for a desired dwell time during which time the therapeutic agent is applied to the targeted location.

A non-perforated irrigating shaped form can be positioned with the drug delivery structure within a body location, such that the irrigating shaped form delivers the first agent from the first agent source to the therapeutic agent delivery structure for interaction with the second agent to form the therapeutic agent and emit out through a portion of the therapeutic agent delivery structure to induce a localized therapeutic effect. The dwell time can be controllable to vary a dosage of therapeutic agent applied to the targeted location. The therapeutic agent delivery system can apply a pressure against the targeted location in a controlled manner including a therapeutic agent fluid pressure for the dwell time, effecting a concentration of the therapeutic agent as the therapeutic agent is applied to the targeted location.

In accordance with an example, a therapeutic agent delivery system includes a therapeutic agent delivery structure completely encapsulated within microporous film. A first agent source contains a first agent and in fluid communication with the therapeutic agent delivery structure. A second agent source contains a second agent and in fluid communication with the therapeutic agent delivery structure. The first agent and second agent can combine to form a therapeutic agent.

In accordance with examples, the therapeutic agent delivery structure can be a non-perforated irrigating shaped form. The second agent can be disposed at least one of one the therapeutic agent delivery structure and within the therapeutic agent delivery structure. The step of positioning the therapeutic agent delivery structure can include inserting the irrigating shaped form into the patient's body proximate to the targeted location requiring treatment. The step of positioning the therapeutic agent delivery structure can include inserting a catheter including the irrigating shaped form into the patient's body proximate to the targeted location requiring treatment.

In accordance with further examples, the second agent can be disposed in a film arranged on at least a portion of the irrigating shaped form. The second agent can be ingressed into the irrigating shaped form. The second agent can be introduced by ingressing the second agent into the irrigating shaped form and through at least a portion of the irrigating shaped form to the patient's body. The first agent can react with the second agent by polymerizing with the second agent to form the therapeutic agent as the first agent and the second agent passing through the plurality of locations to the patient's body.

For completeness reference is made to the following publications:
WO03/009883 discloses a catheter for use in delivering formulation that allows modification of the formulation prior to or concomitant with its transport or at a delivery site. The catheter comprises an elongate body, which defines an inner lumen extending between the proximal and distal ends, and a modifying element, which provides for modification of one or more components of a formulation prior to or concomitant with release at the delivery site.
WO01/28606 discloses apparatus and methods for delivery of a drug or compound into a fluid flowing within a tube or catheter. In one embodiment a catheter includes an inner surface coated with a polymer matrix such as a hydrogel. The hydrogel includes captured within it a therapeutic agent. The method of capture may be by various means, including photolabile bonds between the therapeutic agent and the hydrogel. The therapeutic agent is released from the hydrogel by the application of energy to the hydrogel, such as by a laser emitting a wavelength which resonates and breaks the photolabile bond. The released therapeutic agent diffuses out of the hydrogel into the liquid flowing within a lumen of the catheter.
WO00/43501 discloses a radially expandable fluid delivery device for delivering a fluid to a treatment site within the body is disclosed. The fluid delivery device is constructed of a microporous, biocompatible fluoropolymer material having a microstructure that can provide a controlled, uniform, low-velocity fluid distribution through the walls of the fluid delivery device to effectively deliver fluid to the treatment site without damaging tissue proximate the walls of the device. The fluid delivery device includes a tubular member defined by a wall having a thickness transverse to the longitudinal axis of the tubular member and extending between an inner and an outer surface. The wall is characterized by a microstructure of nodes interconnected by fibrils. The tubular member is deployable from a first, reduced diameter configuration to a second, increased diameter configuration upon the introduction of a pressurized fluid to the lumen. The tubular member includes at least one microporous portion having a porosity sufficient for the pressurized fluid to permeate through the wall. Substantially all of the nodes within the microporous portion are oriented such that spaces between the nodes form micro-channels extending from the inner surface to the outer surface of the wall.

### Brief Description of Drawings

The present invention will become better understood with reference to the following description and accompanying drawings, wherein:
**FIG. 1** is a side elevational view in cross-section of a radially expandable device according to the teachings of the present invention, illustrating the device in a first, reduced diameter configuration;
**FIG. 2** is a side elevational view in cross-section of the radially expandable device of **FIG. 1**, illustrating the device in a second, increased diameter configuration;
**FIG. 3** is a schematic representation of the microstructure of a section of the wall of an expanded fluoropolymer irrigating shaped form used during the manufacturing process of the present invention to yield the radially expandable device of the present invention;
**FIG. 4** is diagrammatic illustration of a therapeutic drug delivery system according to one aspect of the present invention;
**FIGS. 5A****,** **5B,** and **5C** are cross-sectional illustrations of the expandable device at the internal wall of a body lumen, according to one aspect of the present invention;
**FIGS. 6A, 6B,** and **6C** are perspective illustrations of stents for use in conjunction with the present invention;
**FIG. 7** is a flow chart illustrating an example method of applying a therapeutic drug according to one aspect of the present invention;
**FIG. 8** is a flow chart illustrating an example method of forming a polymeric body, according to one aspect of the present invention; and
**FIG. 9** is a flow chart illustrating example embodiment of applying a therapeutic gas to a targeted location within a patient's body.

### DETAILED DESCRIPTION

An illustrative embodiment of the present invention relates to a device and system, for combining two or more components within or just prior to introduction to a delivery device for providing a resulting therapeutic agent to a targeted location within a patient. A component can be a slurry of nanoparticles, solid, semi-solid, gel, liquid, or gas that is designed to be mixed together with any combination of another slurry of nanoparticles, solid, semi-solid, gel, liquid, or gas to create a desired therapeutic agent. There can be two or more components required for combination to form the desired therapeutic agent. The mixing of two or more components just prior to delivery to a patient enables the use of certain components and/or agents that would otherwise not be usable because of a relatively short usable life span. It should, however, be noted that the present invention is not limited to use only with components, agents, or drugs with a relatively short life span. Rather the present invention is useful for any therapeutic agent that requires some form of mixing preparation just prior to or simultaneous to localized tissue administration.

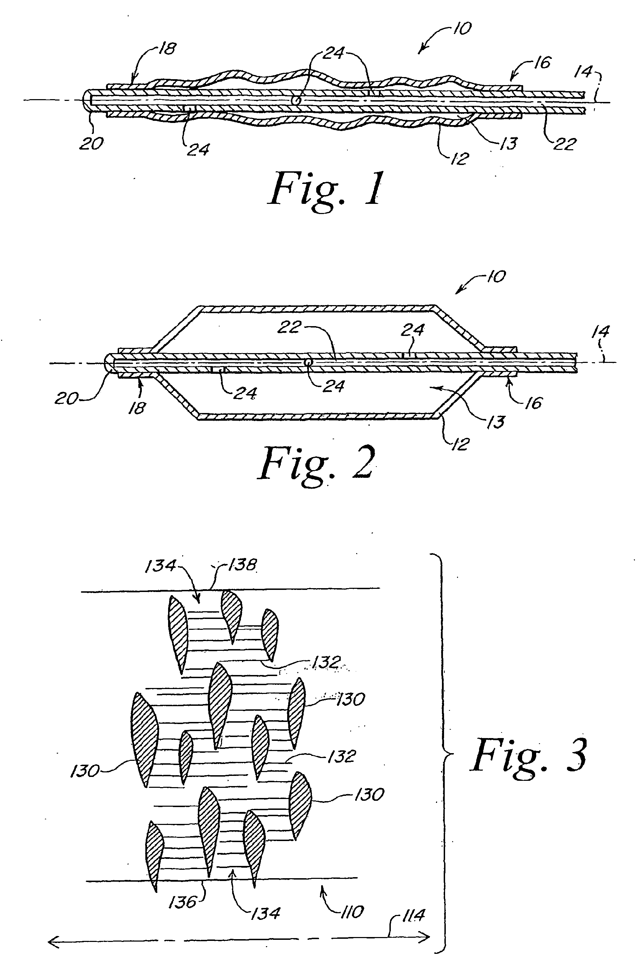

**FIGS. 1** through **9**, wherein like parts are designated by like reference numerals throughout, illustrate an example embodiments of devices and systems for forming and delivering therapeutic elements to a targeted location within a patient formed of at least two components mixed together just prior to entry into the patent, according to the present invention. Although the present invention will be described with reference to the example embodiments illustrated in the figures, it should be understood that many alternative forms can embody the present invention.

A radially expandable device 10 having a shaped form useful for localized tissue irrigation, such as body 12 constructed of a generally inelastic, expanded fluoropolymer material, is illustrated in **FIGS. 1** and **2**. Expandable devices provided by the present invention are suitable for a wide range of applications including, for example, a range of medical treatment applications. Exemplary biological applications include use as a catheter balloon for treatment of implanted vascular grafts, stents, prosthesises, or other type of medical implant, and treatment of any body cavity, space, or hollow organ passage(s) such as blood vessels, the urinary tract, the intestinal tract, nasal cavity, neural sheath, bone cavity, kidney ducts, etc. Additional examples include as a device for the removal of obstructions such as emboli and thrombi from blood vessels, as a dilation device to restore patency to an occluded body passage as an occlusion device to selectively deliver a means to obstruct or fill a passage or space, and as a centering mechanism for transluminal instruments and catheters. The expandable device 10 can also be used as a sheath for covering conventional catheter balloons to control the expansion of the conventional balloon.

The body 12 of the radially expandable device 10 is deployable upon application of an expansion force from a first, reduced diameter configuration, illustrated in **FIG. 1****,** to a second, increased diameter configuration, illustrated in **FIG. 2****.** The radially expandable device 10 of the embodiments illustrated herein can take a number of different irrigating shaped forms. As shown, the expandable member 10 is an expandable irrigating shaped form that can be coupled with a catheter or other structure able to provide fluid (in the form of a slurry of nanoparticles, semi-solid, solid, gel, liquid or gas) to the irrigating shaped form under pressure.

The body 12 of the radially expandable device 10 preferably features a non-perforated monolithic construction, i.e., the body 12 is a singular, unitary article of generally homogeneous material. The example body 12 is manufactured using an extrusion and expansion process described in detail in US Patent Application No. 10/131396, filed April 22, 2002, which is hereby incorporated herein by reference. Alternative methods can include use of plasma treated PTFE, and PTFE stretched with additional wetting as described in US Patent Application No. 09/678,765 filed October 3, 2000. The process yields a body 12 characterized by a seamless construction of inelastic, expanded fluoropolymer. The fluoropolymer has a predefined size and shape in the second, increased diameter configuration. The body 12 can be dependably and predictably expanded to the predefined, fixed maximum diameter and to the predefined shape independent of the expansion force used to expand the device. Alternatively, it should be noted that the aforementioned methods of manufacture relate to the creation of an elastomeric irrigating shaped form suitable for illustrative purposes as an example therapeutic delivery device. The radially expandable device 10 can be made of a number of other different materials as well, as understood by one of ordinary skill in the art. Additional materials that can be utilized with the present invention include a porosity characteristic sufficient to enable fluid to flow therethrough as further described below.

For example, suitable fluoropolymer materials include polytetrafluoroethylene ("PTFE") or copolymers of tetrafluoroethylene with other monomers may be used. Such monomers include ethylene, chlorotrifluoroethylene, perfluoroalkoxytetrafluoroethylene, or fluorinated propylenes such as hexafluoropropylene. PTFE is utilized most often. Accordingly, while the radially expandable device 10 can be manufactured from various fluoropolymer materials, and the manufacturing methods of the present invention can utilize various fluoropolymer materials, the description set forth herein refers specifically to PTFE.

The present invention, therefore, is not limited to using only the elastomeric expandable irrigating shaped form used in the illustrative embodiments of the present disclosure, but can make use of a number of different fluid application device technologies and materials as understood by one of ordinary skill in the art.

Referring specifically to **FIG. 2****,** the body 12 of the radially expandable device 10 is preferably generally tubular in shape when expanded, although other cross-sections, such as rectangular, oval, elliptical, or polygonal, can be utilized. The cross-section of the body 12 is preferably continuous and uniform along the length of the body. However, in alternative embodiments, the cross-section can vary in size and/or shape along the length of the body. **FIG. 1** illustrates the body 12 relaxed in the first, reduced diameter configuration. The body 12 has a central lumen 13 extending along a longitudinal axis 14 between a first end 16 and second end 18.

A deployment mechanism in the form of an elongated hollow tube 20 is shown positioned within the central lumen 13 to provide a radial deployment or expansion force to the body 12. The radial deployment force effects radial expansion of the body 12 from the first configuration to the second increased diameter configuration illustrated in **FIG. 2****.** The first end 16 and the second end 18 are connected in sealing relationship to the outer surface of the hollow tube 20. The first and second ends 16 and 18 can be thermally bonded, bonded by means of an adhesive, or attached by other means suitable for inhibiting fluid leakage from the first and second ends 16 and 18 between the walls of the body 12 and the tube 20.

The hollow tube 20 includes an internal, longitudinal extending lumen 22 and a number of side-holes 24 that provide for fluid communication between the exterior of the tube 20 and the lumen 22. The tube 20 can be coupled to a fluid source or sources (as later described) to selectively provide fluid to the lumen 13 of the body 12 through the lumen 22 and side-holes 24. The pressure from the fluid provides a radially expandable force on the body 12 to radially expand the body 12 to the second, increased diameter configuration. Because the body 12 is constructed from an inelastic material, uncoupling the tube 20 from the fluid source or otherwise substantially reducing the fluid pressure within the lumen 13 of the body 12, does not generally result in the body 12 returning to the first, reduced diameter configuration. However, the body 12 will collapse under its own weight to a reduced diameter. Application of negative pressure, from, for example, a vacuum source, can be used to completely deflate the body 12 to the initial reduced diameter configuration.

One skilled in the art will appreciate that the radially expandable device 10 is not limited to use with deployment mechanisms employing a fluid deployment force, such as hollow tube 20. Other known deployment mechanisms can be used to radially deploy the radially expandable device 10 including, for example, mechanical operated expansion elements, such as mechanically activated members or mechanical elements constructed from temperature activated materials such as nitinol.

**FIG. 3** is a schematic representation of the microstructure of the walls of an ePTFE irrigating shaped form 110, such as the body 12, as formed by an extrusion and expansion process. For purposes of description, the microstructure of the irrigating shaped form 110 has been exaggerated. Accordingly, while the dimensions of the microstructure are enlarged, the general character of the illustrated microstructure is representative of the microstructure within the irrigating shaped form 110.

The microstructure of the ePTFE irrigating shaped form 110 is characterized by nodes 130 interconnected by fibrils 132. The nodes 130 are generally oriented perpendicular to the longitudinal axis 114 of the irrigating shaped form 110. This microstructure of nodes 130 interconnected by fibrils 132 provides a microporous structure having microfibrillar spaces that define through-pores or channels 134 extending entirely from the inner wall 136 and the outer wall 138 of the irrigating shaped form 110. The through-pores 134 are perpendicularly oriented (relative to the longitudinal axis 114), internodal spaces that traverse from the inner wall 136 to the outer wall 138. The size and geometry of the through- pores 134 can be altered through the extrusion and stretching process, as described in detail in Applicants' U.S. Patent Application Serial Number 09/411797, filed on October 1, 1999, to yield a microstructure that is impermeable, semi-impermeable, or permeable.

The size and geometry of the through-pores 134 can be altered to form different orientations. For example, by twisting or rotating the ePTFE irrigating shaped form 110 during the extrusion and/or stretching process, the micro-channels can be oriented at an angle to an axis perpendicular to the longitudinal axis 114 of the irrigating shaped form 110. The expandable device 10 results from the process of extrusion, followed by stretching of the polymer, and sintering of the polymer to lock-in the stretched structure of through-pores 134.

The microporous structure of the through pores 134 of the material forming the expandable device 10 enable permeation of the wall of the expandable device 10 without the need for creating perforations in the expandable device 10. The microporous structure of the device enables a more controllable, and more even, distribution of fluid through the walls of the expandable device 10 relative to a perforated device with fluid exiting the device only at the perforations. Thus, the non-perforated structure of the expandable device 10 contributes to the effective distribution of the fluid by the expandable device 10 as described herein.

In accordance with one embodiment, the ePTFE irrigating shaped form 110, and the resultant expandable device 10, has a fine nodal structure that is uniform throughout the cross section and length of the ePTFE irrigating shaped form. The uniform fine nodal structure provides the expandable device 10 with improved expansion characteristics as the expandable device dependably and predictably expands to the second diameter. The fine nodal structure can be characterized by nodes having a size and mass less than the nodes found in conventional ePTFE grafts, for example in the range of 25 µm - 30 µm. Additionally, the spacing between the nodes, referred to as the internodal distance, and the spacing between the fibers, referred to as the interfibril distance, can also be less than found in conventional ePTFE grafts, for example in the range of 1µm - 5 µm. Moreover, the internodal distance and the interfibril distance in the preferred embodiment can be uniform throughout the length and the cross section of the ePTFE irrigating shaped form. The uniform nodal structure can be created by forming the billet with a uniform lubricant level throughout its cross section and length. Stretching the tubular extrudate at higher stretch rates, for example at rates greater than 25,4 mm/s (1 in/s), yields the fine nodal structure. Preferably, the extrudate is stretched at a rate of approximately 25,4 mm/s (10 in/s) or greater. The nodal structure can also be non-uniform, by varying the location and amount of lubrication and stretching processes.

In the instance of the fluid inflating the body 12 of the radially expandable device 10, the fluid can pass through the body 12 in a weeping manner, and be applied to a targeted location in the patient body, as discussed further below. The fluid can be under fluid pressure when contacting the targeted location. The fluid can further contain one or more drugs having therapeutic properties for healing the affected targeted location. Example therapeutic drugs and therapeutic agents can include those listed in Table 1 below.

**Table #1**

| **CLASS** | **EXAMPLES** |
|---|---|
| Antioxidants | Alpha-tocopherol, lazaroid, probucol, phenolic antioxidant, resveretrol, AGI-1067, vitamin E |
| Antihypertensive Agents | Diltiazem, nifedipine, verapamil |
| Antiinflammatory Agents | Glucocorticoids, NSAIDS, ibuprofen, acetaminophen, hydrocortizone acetate, hydrocortizone sodium phosphate |
| Growth Factor Antagonists | Angiopeptin, trapidil, suramin |
| Antiplatelet Agents | Aspirin, dipyridamole, ticlopidine, clopidogrel, GP IIb/IIIa inhibitors, abcximab |
| Anticoagulant Agents | Bivalirudin, heparin (low molecular weight and unfractionated), wafarin, hirudin, enoxaparin, citrate |
| Thrombolytic Agents | Alteplase, reteplase, streptase, urokinase, TPA, citrate |
| Drugs to Alter Lipid Metabolism (e.g. statins) | Fluvastatin, colestipol, lovastatin, atorvastatin, amlopidine |
| ACE Inhibitors | Elanapril, fosinopril, cilazapril |
| Antihypertensive Agents | Prazosin, doxazosin |
| Antiproliferatives and Antineoplastics | Cyclosporine, cochicine, mitomycin C, sirolimus microphenonol acid, rapamycin, everolimus, tacrolimus, paclitaxel, estradiol, dexamethasone, methatrexate, cilastozol, prednisone, cyclosporine, doxorubicin, ranpirnas, troglitzon, valsarten, pemirolast |
| Tissue growth stimulants | Bone morphogeneic protein, fibroblast growth factor |
| Gasses | Nitric oxide, super oxygenated 02 |
| Promotion of hollow organ occlusion or thrombosis | Alcohol, surgical sealant polymers, polyvinyl particles, 2-octyl cyanoacrylate, hydrogels, collagen, liposomes |
| Functional Protein/Factor delivery | Insulin, human growth hormone, estrogen, nitric oxide |
| Second messenger targeting | Protein kinase inhibitors |
| Angiogenic | Angiopoetin, VEGF |
| Anti-Angiogenic | Endostatin |
| Inhibitation of Protein Synthesis | Halofuginone |
| Antiinfective Agents | Penicillin, gentamycin, adriamycin, cefazolin, amikacin, ceftazidime, tobramycin, levofloxacin, silver, copper, hydroxyapatite, vancomycin, ciprofloxacin, rifampin, mupirocin, RIP, kanamycin, brominated furonone, algae byproducts, bacitracin, oxacillin, nafcillin, floxacillin, clindamycin, cephradin, neomycin, methicillin, oxytetracycline hydrochloride. |
| Gene Delivery | Genes for nitric oxide synthase, human growth hormone, antisense oligonucleotides |
| Local Tissue perfusion | Alcohol, H2O, saline, fish oils, vegetable oils, liposomes |
| Nitric oxide Donative Derivatives | NCX 4016 - nitric oxide donative derivative of aspirin, SNAP |
| Gases | Nitric oxide, super oxygenated O₂ compound solutions |
| Imaging Agents | Halogenated xanthenes, diatrizoate meglumine, diatrizoate sodium |
| Anesthetic Agents | Lidocaine, benzocaine |
| Descaling Agents | Nitric acid, acetic acid, hypochlorite |
| Chemotherapeutic Agents | Cyclosporine, doxorubicin, paclitaxel, tacrolimus, sirolimus, fludarabine, ranpirnase |
| Tissue Absorption Enhancers | Fish oil, squid oil, omega 3 fatty acids, vegetable oils, lipophilic and hydrophilic solutions suitable for enhancing medication tissue absorption, distribution and permeation |
| Anti-Adhesion Agents | Hyalonic acid, human plasma derived surgical sealants, and agents comprised of hyaluronate and carboxymethylcellulose that are combined with dimethylaminopropyl, ehtylcarbodimide, hydrochloride, PLA, PLGA |
| Ribonucleases | Ranpimase |
| Germicides | Betadine, iodine, sliver nitrate, furan derivatives, nitrofurazone, benzalkonium chloride, benzoic acid, salicylic acid, hypochlorites, peroxides, thiosulfates, salicylanilide |

Surgical adhesives, anti-adhesion gels and/or films, and tissue-absorbing biological coatings can also be utilized with the present invention and with or without the therapeutic drugs and agents of Table 1. The adhesive-type polymers can include both one and two-part adhesives for use with or without the therapeutic drugs or agents. Examples of the adhesive-type polymers include 2-octyl cyanoacrylate, a patient's own plasma mixed with a suspension of human derived collagen and thrombin to form a natural biological sealant, fibrin glue derived from preparation of the patient's blood, polymeric hydrogels, and the like. The tissue-absorbing therapeutic agents, as shown in Table 1, can be incorporated into the fluid such as those which include fish oil omega 3 fatty acids, vegetable oils containing fish oil omega 3 fatty acids, other oils or substances suitable for enhancing tissue absorption, adhesion, lipophillic permeation, and any combination thereof Anti-adhesion film forming gels, solutions, or compounds can be used with or without therapeutic drugs to enhance tissue adhesion of the agents and improve intra-cellular and extra-cellular therapeutic agent permeation simultaneous to reducing traumatic tissue adhesion formation in and around the targeted treatment site. Reduced tissue adhesion formation in selected areas prone to adhesion formation, such as stented vessels, dilated urethras, and the like, benefit from such an anti-adhesion therapeutic delivery method.

The internodal distance and the interfibral distance can be varied to control over a relatively larger range, to allow a fluid to pass through the through-pores or channels 134. The size of the through-pores or channels 134 can be selected through the manufacturing process, for example as described in detail in US Patent Application No. 09/411797. The internodal distance of microstructure of the wall within the microporous region, and hence the width of the through-pores or channels 134, can be approximately 1µm to approximately 150µm. Internodal distances of this magnitude can yield flow rates of approximately 0.01 ml/min to approximately 100 ml/min of fluid through the wall of the body 12.

The internodal distances can also vary at different locations along the microporous structure to result in the channels 134 being of different sizes in different locations or regions. This enables different flow rates to occur through different areas of the same microporous structure at a substantially same fluid pressure.

The different flow rates achieved by the radially expandable device 10 can contribute to variations in fluid pressure during inflation of the expandable device 10, and also enable a variation in dwell time of the expandable device 10 at a targeted location requiring therapeutic treatment. An additional factor can include the relative viscosity of the fluid(s) to each other for mixing purposes, and the resulting fluid viscosity of the therapeutic agent. The more viscous, the more resistant to flow, thus the longer dwell time required to apply a sufficient amount of agent.

Dwell time is a measurement of the amount of time the expandable device 10 is disposed within the patient body applying one or more therapeutic agents to a location within the patient body, such as a targeted location. The targeted location is a location requiring therapeutic treatment. The ability to vary the size and shape of the through-pores or channels 134 enables modification of the dwell time. If a longer dwell time is desired, the size and shape of the through-pores 134 can be varied to allow less fluid to pass through. Likewise, if a shorter dwell time is desired with the same amount of therapeutic fluid to be applied, the through-pores 134 can be varied to allow more fluid to pass through at a faster rate. In addition, the dwell time can be affected by the pressurization of the fluid being absorbed by the tissue of the body lumen in accordance with one example embodiment of the present invention and later described herein.

The microporous structure of the through-pores 134 is such that the fluid pressure of the fluid passing through can vary over a substantial range and still result in substantially the same rate of fluid flow through the through-pores 134. For example, for a predetermined range of fluid pressures, the rate of fluid flow through the through-pores 134 remains substantially constant for a given embodiment. Alternatively, the percentage of change of the rate of fluid flow can be made less than a given percentage of change of fluid pressure. The pressure within the expandable device 10 can range, for example in one embodiment involving the pressurization of the fluid external to the expandable device 10, up to or at about six atmospheres. Other ranges that have been shown to work with the expandable device 10 include pressures in the range of two atmospheres to four atmospheres. One result of having relatively lower fluid pressure within the flexible expandable device 10 is that the expandable device 10 is able to conform to the shape of the body lumen or cavity within which the expandable device 10 operates, rather than the expandable device 10 causing trauma to the body tissue from over-expansion.

The pressure within the expandable device 10 can be supplied in a constant, variable, or intermittent amount by varying the flow of fluid to the expandable device 10. The variation of fluid pressure inside the expandable device 10 can influence a variation of the fluid pressure external to the expandable device 10 as described further below.

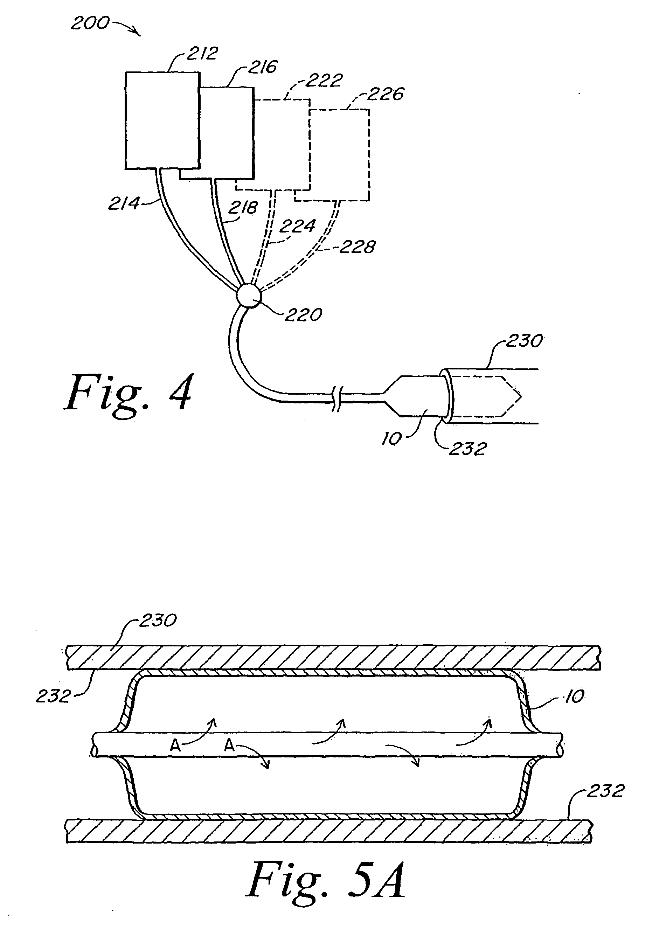

In accordance with the teachings of the present invention, FIG. 4 illustrates a therapeutic drug delivery system 200. The expandable device 10 is in fluid communication with a first storage container 212 through a tubular coupling 214. The expandable device 10 is also in fluid communication with a second storage container 216 through a second tubular coupling 218. Different amounts of a component or components in fluid form from the first storage container 212 and the second storage container 216 can be mixed together within the expandable device 10 prior to exit from the expandable device 10 and entry into the patient. In addition, the coupling with the expandable device 10 is removable to switch connections to storage containers easily.

There can be a number of additional storage containers represented by storage container 222 with tubular coupling 224 and storage container 226 with tubular coupling 228. The number of storage containers 212, 216, 222, and 226 (and corresponding tubular couplings 214, 218, 224, and 228) is determined by the number of components required to be maintained separately until the desired mixing process occurs. Each storage container 212, 216, 222, and 226 can maintain a separate component until mixing occurs. Therefore, the number of storage containers can vary. In addition, the type of storage container can vary. Any of the storage containers 212, 216, 222, and 226 can be suitable for holding a solid, liquid, or gas. More specifically, the first storage container 212 can be designed to hold a liquid, while the second storage container 216 can be designed to hold a gas, or vice versa, or one or the other could hold another of the solids, liquids, or gases. It is not necessary for any single container design to be able to hold solids, liquids, and/or gases, but such a design would be functional with the present invention.

Alternatively, different designs can be provided depending on the physical state of the component being stored. The solid that can be held by the storage containers 212, 216, 222, and 226 can be in powder form, such that the solid can be easily transferred to the expandable device 10 for mixing with a liquid or gas. Further, the storage containers 212, 216, 222, and 226 can be heated or cooled to maintain a desired temperature of the component being stored, if necessary.

For the remainder of this description, the examples discussed will make use of the first storage container 212 and the second storage container 216. However, it should be appreciated that the Applicants are referring to the storage containers 212, 216, 222, and 226, and additional containers not numbered, as a plurality when referring to the first and second storage containers 212 and 216. Thus, any number of storage containers required for a specific embodiment, from one to a plurality, is considered to be anticipated by the present two-container description and illustrations.

A controller 220 can be included along the first tubular coupling 214 and the second tubular coupling 218 vary or control the amount of component fluid passing through to the expandable device 10. The controller 220 can take a number of different forms. Primarily, the controller 220 restricts flow and/or diverts flow from the first and second storage containers 212 and 216, and any additional containers. The controller 220 can include a simple valve with adjustable flow rates, or can be more elaborate as understood by one of ordinary skill in the art. The example controller can also introduce sufficient pumping action to pressurize the fluid supplied by the first and second storage containers 212 and 216 to the expandable device 10. Alternatively, the storage containers 212 and 216 themselves can be pressurized. An example controller is a pressure infusor conventionally employed for angioplasty balloon catheter inflation with a pressure gauge. One ore more pressure infusor devices connected to a manifold provides multiple therapeutic element infusion into the device.

The first storage container 212 can contain a component fluid that is different from the component fluid in the second storage container 216. The component fluids in each of the first storage container 212 and the second storage container 216 can contain any number of therapeutic agents or other liquids or gases as desired. The therapeutic drug delivery system 200 is useful when the component fluids in each of the first storage container 212 and the second storage container 216 generate a chemical, physical, polymeric, lilophilic, water soluble, lipidphilic, non-water soluble, or other, reaction or process when mixed together. The reaction generally creates a fluid that either has a relatively short life span, or changes properties relatively quickly (such as in a number of minutes or hours) so that it is difficult to store such a mixture and ship it to clinical users without the mixture becoming ineffective or unusable. The resulting fluid can also maintain improved therapeutic benefits for a limited time period, as well. Thus, to obtain the most benefits from the mixture, each of the components of the mixture (i.e., the component fluids stored in each of the storage containers 212 and 216) must be mixed just prior to introduction into the patient. It should be noted that there is no requirement for the mixture to have a relatively short useable life span, or any other characteristic that would require the creation of the mixture just prior to use. The mixture can be mixed by the therapeutic drug delivery system with the resultant mixture having a usable life of, e.g., days, weeks, or years. However, the more common application of the therapeutic drug delivery system of the present invention is likely for mixtures having a shorter usable life span. Further, the mixture of two or more components is not merely the combination of two different therapeutic agents that otherwise can be administered separately and without requirement of being mixed together. The components that are mixed together with the method of the present invention result in a therapeutic agent, or a therapeutic agent that is enhanced or improved as a result of the mixture.

In an alternative arrangement, the first tubular coupling 214 and the second tubular coupling 218 can feed to the expandable device 10 without the interjection of the controller 220. The amounts of the fluids necessary for the mixture can be determined by the amount of dilution (or lack thereof) for each fluid separately.

Additional examples include variation in the source and location of two or more components to create the therapeutic drug or agent. The components can each reside in separate storage containers as discussed above. One of the components can reside in or on the expandable device 10 and mix with other components as the other components enter the expandable device 10, or pass through the walls of the expandable device 10. In the present invention one component originates in a storage container. Another, second component exists in a coating on the expandable device, or as a part of the PTFE or other material forming the expandable device 10. As the component in the storage container passes through the walls of the expandable device 10, the component mixes with the second component on the expandable device 10, to form the therapeutic drug or agent just prior to delivery to the patient. The component on the expandable device 10 can further be in the form of an adhesive, or the like.

More specifically, the components that are mixed together to form the therapeutic drug or agent can, in accordance with one embodiment, include a two-part adhesive. As each of the components of the two-part adhesive mix together, the adhesive fluid forms. The adhesive fluid then passes through the expandable device 10 or 210 to the patient, where the adhesive is applied and cures in place simultaneous to irrigating shaped form inflation. The same pressure controller deflates the expandable device 10 or 210 via negative pressure applied to the fluid just prior to the time dependent adhesive curing.

The adhesive can also be utilized in applying one or more components to the surface of the expandable device. As additional components are supplied through the therapeutic drug delivery system, they combine and mix with the adhesive and component or components disposed with the adhesive, and the desired therapeutic drug results.

Whether there are multiple components in the storage containers 212 and 216, or single components, and whether the components are in solid, liquid, or gas form, various characteristics of the components can be changed. For example, the components can be diluted or strengthened, heated or cooled, mixed or layered, and the like. In addition, the components can be varied in terms of their supply, e.g., constant, variable, or intermittent flow rates can be provided to the expandable device 10 and through the expandable device 10. Further, the components can be varied in terms of state, e.g., solid powder, semi-solid, nanoparticles, gel, liquid, gaseous, highly viscous liquid, cured coating, intermixed with a polymer such as PTFE, and the like.

In accordance with further examples the one or more components can be combined to form a polymeric body with or without a therapeutic agent. For example, the storage containers 212 and 216 can each contain components that when combined, create a polymer material. Upon delivery of the first component and the second component to the expandable device 10, the components mix and then emit through to a targeted location within the patient. At the targeted location, the mixture cures to form the polymeric structure. Such a structure can be used to seal internal hemorrhages, cover a set of stitches to create a smooth surface, bond body tissues together, coat a diseased or damaged tissue with a protective coating, and the like.

It should be noted that the resulting agent, whether therapeutic or non-therapeutic, can have the physical form including a gas, liquid, powder, gel, micro-particle, and nano-particle.

The expandable device 10 is shown inserted into a partial sectional representation of a body lumen 230 having an internal wall 232 in **FIG. 5A****.** The body lumen 230 can be, for example, a blood vessel, capillary, or other enclosed structure into which the expandable device 10 can be inserted. Application of the expandable device 10 is discussed further below.

In operation, the expandable device 10 is inserted into the patients body and maneuvered to the targeted location, for example, in the body lumen 230 shown in **FIG. 4****.** The pressure within the expandable device 10 can range over a number of different pressures as understood by one of ordinary skill in the art. For example, the pressure can range up to about six atmospheres in one example embodiment, between about two atmospheres and about four atmospheres according to another example, or another desired range of pressure. The expandable device 10 can inflate, under pressure from an ingressing fluid or agent, to push against the internal wall 232 of the body lumen 230 in which the expandable device 10 is implanted. It should again be noted that the blood vessel representing the body lumen 230 is merely an illustrative example of an appropriate targeted location for introduction of therapeutic agents by the expandable device 10 in accordance with the present invention.

The expandable device 10 is provided in a number of different size ranges, such that the size of the expandable device 10 in fully expanded state is greater than 100% of the inner diameter size of the body lumen or cavity in which the expandable device 10 is placed. In other words, the expandable device 10 inflates and takes up sufficient space within the body lumen or cavity to create a pressure applied by the expandable device 10 against the tissue of the body lumen or cavity. If the expandable device 10 is too small, when it is fully expanded it will not reach the walls of the body lumen, and therefore no contact will be created. If the expandable device 10 is too large, full expansion of the device 10 will cause trauma to the body lumen or cavity. In some instances, this may be desirable (if the desire is to force the healing repair of a vessel, for example). However, in other instances, an expandable device 10 too large for the body lumen or cavity is undesirable. Therefore, the user must select a size appropriate for the task at hand. For example, for the situation where the user requires that the expandable device 10 apply a non-traumatic pressure to the body lumen or cavity, the expandable device 10 can be selected to expand to about 101 % to 150% of the inner diameter of the body lumen or cavity. Other size ranges are possible, based on pressure applied to the expandable device 10, strength of the body lumen or cavity, and desire for non-traumatic or traumatic results, as understood by one of ordinary skill in the art.

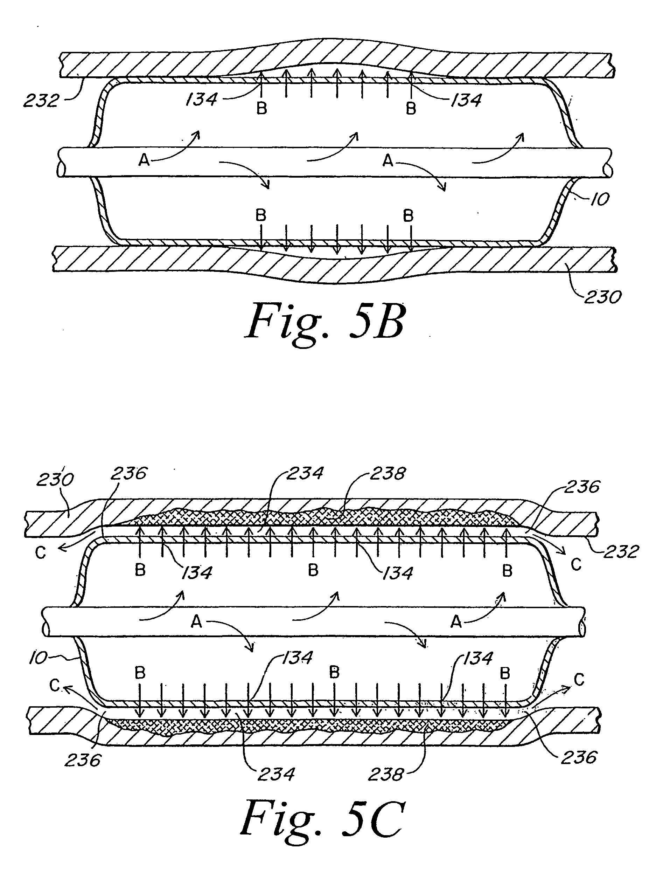

The pressure placed by the expandable device 10 on the internal wall 232 can create a semi-confined space 234 in accordance with one example embodiment as illustrated in **FIG. 5C****.** The semi-confined space 234 can be defined as the area between the expandable device 10 as the expandable device 10 is pressed against the internal wall 232 of the body lumen 230. The semi-confined space 234 is bordered on one side by the expandable device 10, on an opposite side by the internal wall 232 of the body lumen, and on a third side by a small orifice 236 around the edges of the expandable device 10 where the expandable device ends as the pressurized fluid occupies the space.

To further elaborate, **FIG. 5A** shows the expandable device 10 inflated via the fluid flowing in the direction of arrows A and pressed against the internal wall 232 of the body lumen 230. In the illustrated state, there is no semi-confined space 234 because the fluid that is expanding the expandable device 10 has not yet passed through the walls of the expandable device. Once sufficient fluid has passed through the walls of the expandable device, the fluid remains pressurized and pushes against the internal wall 232 and the outside wall of the expandable device 10 to form the semi-confined space 234. **FIG. 5B** illustrates some additional fluid gathering external to the expandable device 10 and beginning to form the semi-confined space 234 (however, the space has not been completed as shown). Additional pressurized fluid provided external to the expandable device 10 expands the space to form the semi-confined space 234 as shown in **FIG. 5C****.** Once complete, the semi-confined space 234 reaches the end of the expandable device 10 and the small orifice 236 is created. With additional pressurized fluid provided to the expandable device 10, the pressure external to the expandable device 10 is maintained, the semi-confined space 234 is maintained, and the small orifice 236 remains open. If the pressure of the fluid external to the expandable device falls substantially, then the small orifice 236 will close.

The semi-confined space 234 channels the pressurized fluid emitting through the through-pores 134 of the expandable device 10 in the direction of the arrows B shown. This arrangement causes the therapeutic agents and/or drugs concentrated in the fluid to have complete exposure to the targeted location of the internal wall 232. As such, at least some of the therapeutic agents and/or drugs permeate into the localized cellular space and tissue of the internal wall 232 into a permeation region 238. In addition, some of the fluid creates and then leaks out through the small orifice 236 around the edges of the expandable device 10 in the direction of arrows C. Thus, some of the pressure from within the expandable device 10 carries through to the semi-confined space 234, resulting in the fluid being pressurized against the internal wall 232 of the body lumen 230. Once the fluid exits the semi-confined space 234, the drugs and/or agents contained within the fluid are diluted and subsequently washed away. This process is termed the kinetic isolation pressurization (KIP) effect.

The KIP effect is instrumental in creating the semi-confined space 234 between the expandable device 10 and the internal wall 232 of the body lumen 230, and thus creating a more even distribution or deposition of therapeutic drug or agent at the permeation region 238 of the internal wall 232. This semi-confined space 234 is continuously filled with fluid passing through the wall of the expandable device 10 and feeding into the semi-confined space 234. With the continuous fluid movement, and the elevated pressure within the semi-confined space 234, the actual structure of the expandable device 10 does not maintain contact with the internal wall 232 or the permeation region 238 for any extended period. Therefore, a continually churning volume of fluid containing a concentration of at least one therapeutic agent or drug is deposited at the internal wall 232. There is no opportunity for some areas of therapeutic drug or agent to become stagnated in a location on the tissue of the internal wall 232 because the fluid movement constantly chums the therapeutic drug or agent, continually providing a fresh supply and even or substantially uniform deposition.

The continuous churning and re-supply of the fluid containing the at least one therapeutic drug or agent provides a regulated, substantially uniform, therapeutic drug or agent concentration at the tissue. The pressurized fluid also provides for atraumatic delivery or deposition of the therapeutic drugs or agents. Further, there is no structural impediment to drug deposition, such as struts from a stent, or areas of compression by a balloon against the internal wall 232, that may cause pooling of the fluid and thus the therapeutic drug or agent. With an even deposition of a substantially uniform concentration of therapeutic agent or drug, there is an increased efficiency in tissue permeation, and a more even concentration of therapeutic drug or agent permeating the internal wall 232 of the body lumen 230.

The delivery of a therapeutic agent or drug must achieve sufficient concentration at the targeted location for efficacy. Prior methods required use of a substantially higher dosemetric or volumetric amount of drug or agent to attempt to achieve a therapeutic effect at the targeted location relative to the present invention. Prior methods had to include sufficient amounts of a drug or agent to permeate the tissue while also working around structures such as stent struts, and while being washed away from the targeted location. Alternatively, prior methods supplied a substantially greater amount of drug to a patient using a systemic approach rather than a targeted approach. However, the present invention provides an atraumatic method of increasing permeation of tissue by at least one therapeutic drug and/or agent using a pressurized fluid more concentrated with the therapeutic drug and/or agent for a more efficient and uniform distribution of the therapeutic drug and/or agent to the tissue of the targeted location.

Numerous modifications and alternative embodiments of the present invention will be apparent to those skilled in the art in view of the foregoing description. Accordingly, this description is to be construed as illustrative only and is for the purpose of teaching those skilled in the art the best mode for carrying out the present invention.

## Claims

1. A therapeutic agent delivery system, comprising:
a non-perforated irrigating shaped form (10) in fluid communication with a first agent source (212), the irrigating shaped form sized and dimensioned for positioning within a patient's body; and
a second agent disposed in a coating on the irrigating shaped form;
**characterized in that:**
the non-perforated irrigating shaped form has a microporous structure extending between an inner wall (136) and an outer wall (138) of the non-perforated irrigating shaped form;
the non-perforated irrigating shaped form is sized and dimensioned for positioning within a patient's body against a targeted location (238); and
wherein upon delivery of a first agent from the first agent source permeating through the microporous structure of the irrigating shaped form, the first agent reacts with the second agent forming the therapeutic agent on a portion (134) of the irrigating shaped form for a desired dwell time.

2. The therapeutic agent delivery system of claim 1, wherein a fluid pressure and dwell time of the therapeutic agent delivery system are controllable to vary permeability of the therapeutic agent into the targeted location.

3. The therapeutic agent delivery system of claim 1, wherein the irrigating shaped form is structurally suitable for pressurization at about 6 atmospheres.

4. The therapeutic agent delivery system of claim 1, wherein a physical state if the therapeutic agent comprises at least one of a gas, liquid, powder, gel, micro-particle, and nano-particle.

5. The therapeutic agent delivery system of claim 1, wherein the first agent and the second agent have substantially similar viscosity.

6. The therapeutic agent delivery system of claim 1, wherein the therapeutic agent emits to the targeted location under pressure and maintains fluid pressure external to the irrigating shaped form for the desired dwell time.

7. The therapeutic delivery structure of claim 1, wherein the dwell time is controllable to vary a dosage of the therapeutic agent applied to the targeted location.

8. The therapeutic delivery system of claim 1, wherein the non-perforated irrigating shaped form has a lumen (13) extending along a longitudinal axis (14) of the form; and the non-perforated irrigating shaped form is radially expandable with respect to the axis.

9. The therapeutic delivery system of claim 1, wherein the non-perforated irrigating shaped form is made from an inelastic material.

10. The therapeutic agent delivery system of claim 1, wherein the irrigating shaped form is coupled with the first agent source.

## Patentansprüche

1. Therapeutikumabgabesystem, umfassend:
eine nicht perforierte, zum Spülen geformte Form (10) in Strömungsverbindung mit einer ersten Wirkstoffquelle (212), wobei die zum Spülen geformte Form zum Anordnen innerhalb eines Körpers eines Patienten bemessen und ausgelegt ist; und
einen zweiten Wirkstoff, der in einer Beschichtung auf der zum Spülen geformten Form angeordnet ist;
**dadurch gekennzeichnet, dass:**
die nicht perforierte, zum Spülen geformte Form eine mikroporöse Struktur aufweist, die sich zwischen einer inneren Wand (136) und einer äußeren Wand (138) der nicht perforierten, zum Spülen geformten Form ausdehnt;
die nicht perforierte, zum Spülen geformte Form zum Anordnen innerhalb des Körpers eines Patienten an einer vorherbestimmten Stelle (238) bemessen und ausgelegt ist; und
wobei nach Abgabe eines ersten Wirkstoffs aus der ersten Wirkstoffquelle, der durch die mikroporöse Struktur der zum Spülen geformten Form dringt, der erste Wirkstoff mit dem zweiten Wirkstoff reagiert, wodurch das Therapeutikum auf einem Abschnitt (134) der zum Spülen geformten Form für eine gewünschte Einwirkdauer ausgebildet ist.

2. Therapeutikumabgabesystem nach Anspruch 1, wobei ein Flüssigkeitsdruck und eine Einwirkdauer des Therapeutikumabgabesystems zum Variieren der Permeabilität des Therapeutikums in die vorherbestimmte Stelle regelbar sind.

3. Therapeutikumabgabesystem nach Anspruch 1, wobei die zum Spülen geformte Form strukturell zur Druckbeaufschlagung von ungefähr 6 Atmosphären geeignet ist.

4. Therapeutikumabgabesystem nach Anspruch 1, wobei ein physikalischer Zustand des Therapeutikums zumindest eines von einem Gas, einer Flüssigkeit, einem Pulver, einem Gel, einem Mikropartikel und einem Nanopartikel umfasst.

5. Therapeutikumabgabesystem nach Anspruch 1, wobei der erste Wirkstoff und der zweite Wirkstoff im Wesentlichen eine ähnliche Viskosität aufweisen.

6. Therapeutikumabgabesystem nach Anspruch 1, wobei das Therapeutikum unter Druck zu der vorherbestimmten Stelle ausströmt und einen Flüssigkeitsdruck außerhalb der zum Spülen geformten Form für die gewünschte Einwirkdauer beibehält.

7. Therapeutikumabgabesystem nach Anspruch 1, wobei die Einwirkdauer zum Variieren einer Dosis des Therapeutikums, die der vorherbestimmten Stelle zugeführt ist, regelbar ist.

8. Therapeutikumabgabesystem nach Anspruch 1, wobei die nicht perforierte, zum Spülen geformte Form einen inneren Raum (13) aufweist, das entlang einer Längsachse (14) der Form verläuft; und die nicht perforierte, zum Spülen geformte Form bezüglich der Achse radial ausdehnbar ist.

9. Therapeutikumabgabesystem nach Anspruch 1, wobei die nicht perforierte, zum Spülen geformte Form aus einem unelastischen Material hergestellt ist.

10. Therapeutikumabgabesystem nach Anspruch 1, wobei die zum Spülen geformte Form mit der ersten Wirkstoffquelle verkuppelt ist.

## Revendications

1. Un système d'apport d'agent thérapeutique, comprenant :
un dispositif non perforé de forme adaptée à l'irrigation (10) en communication fluide avec une source de premier agent (212), le dispositif de forme adaptée à l'irrigation étant calibré et dimensionné pour un positionnement à l'intérieur d'un corps d'un patient ; et
un second agent disposé dans un revêtement sur le dispositif de forme adaptée à l'irrigation ;
**caractérisé en ce que :**
le dispositif non perforé de forme adaptée à l'irrigation a une structure microporeuse s'étendant entre une paroi intérieure (136) et une paroi extérieure (138) du dispositif non perforé de forme adaptée à l'irrigation ; le dispositif non perforé de forme adaptée à l'irrigation est calibré et dimensionné pour un positionnement à l'intérieur du corps d'un patient sur un site ciblé (238) ; et
dans lequel, à l'apport d'un premier agent à partir de la source de premier agent filtrant à travers la structure microporeuse du dispositif de forme adaptée à l'irrigation, le premier agent réagit avec le second agent formant l'agent thérapeutique sur une portion (134) du dispositif de forme adaptée à l'irrigation pour un temps de maintien souhaité.

2. Le système d'apport d'agent thérapeutique de la revendication 1, dans lequel une pression de fluide et un temps de maintien du système d'apport d'agent thérapeutique sont contrôlables pour varier la perméabilité de l'agent thérapeutique dans le site ciblé.

3. Le système d'apport d'agent thérapeutique de la revendication 1, dans lequel le dispositif de forme adaptée à l'irrigation convient structurellement à une pressurisation à environ 6 atmosphères.

4. Le système d'apport d'agent thérapeutique de la revendication 1, dans lequel un état physique de l'agent thérapeutique comprend au moins celui d'un gaz, d'un liquide, d'une poudre, d'un gel, d'une microparticule et d'une nanoparticule.

5. Le système d'apport d'agent thérapeutique de la revendication 1, dans lequel le premier agent et le second agent ont une viscosité considérablement similaire.

6. Le système d'apport d'agent thérapeutique de la revendication 1, dans lequel l'agent thérapeutique se répand vers le site ciblé sous pression et maintient une pression du fluide à l'extérieur du dispositif de forme adaptée à l'irrigation pendant le temps de maintien souhaité.

7. La structure d'apport thérapeutique de la revendication 1, dans laquelle le temps de maintien est contrôlable pour varier un dosage de l'agent thérapeutique appliqué au site ciblé.

8. Le système d'apport thérapeutique de la revendication 1, dans lequel le dispositif non perforé de forme adaptée à l'irrigation a un espace tubulaire (13) s'étendant le long d'un axe longitudinal (14) du dispositif ; et le dispositif non perforé de forme adaptée à l'irrigation est radialement extensible par rapport à l'axe.

9. Le système d'apport thérapeutique de la revendication 1, dans lequel le dispositif non perforé de forme adaptée à l'irrigation est fabriqué à partir d'un matériau inélastique.

10. Le système d'apport d'agent thérapeutique de la revendication 1, dans lequel le dispositif de forme adaptée à l'irrigation est lié à la source de premier agent.
